# EUROPEAN PATENT APPLICATION

(11) **EP 1 234 591 A2**
(43) Date of publication of application: **28.08.2002**
(21) Application number: 02425086.2
(22) Date of filing: 20.02.2002
(51) Int. Cl.: A61M 11/06, B05B 7/00

(54) **Non-spill nebuliser for aerosol systems**

(30) Priority: 21.02.2001 IT BS20010020 U
(71) Applicant: FLAEM NUOVA S.p.A., I-25010 S. Martino Della Battaglia (Brescia) (IT)
(72) Inventor: Abate, Riccardo, 25010 S.Martino della Battaglia (Brescia (IT)
(74) Representative: Manzoni, Alessandro

(57) **Abstract**

This invention covers a nebuliser (10) for aerosol systems comprised of a non-spill system (25) at the opening (21) conveying the nebulised medication (15) towards the user, thus preventing the liquid medication (15) from spilling if the nebulised is inclined or dropped.

## Description

This invention covers in general aerosol systems for nebulising medication for aerosol therapy purposes.

Various makes and configurations of nebulisers for medication aerosol systems are already known. An example of a nebuliser is descriped in particular in the previous application for a utility model no. BS/97/U/000121 by the same applicant.

Like other known ones, such a nebuliser, however, does not possess a non-spill system. Some standards, however, state the nebulisers for the above-mentioned use must be designed in such a way as not to allow spilling of the liquid medication in the case of excessive inclination, dropping or overturning of the nebuliser.

It is therefore the aim of this invention to design and develop a nebuliser for use in aerosol systems, equipped advantageously with an effective spill-prevention means complying with said standards.

A further aim of this invention is to provide a nebuliser for the above-mentioned use having a particularly simple non-spill system made integral with a component of the nebuliser and which does not effect the normal structure or operation of the nebuliser.

Said aims are, in accordance with the invention, achieved by means of a nebuliser, such as of the type described in the above-mentioned utility model application, having a non-spill septum at the outlet of the nebuliser towards the user.

Further details of the invention will made more evident in the description below with reference to the attached drawings, by a way of non limiting examples, in which:

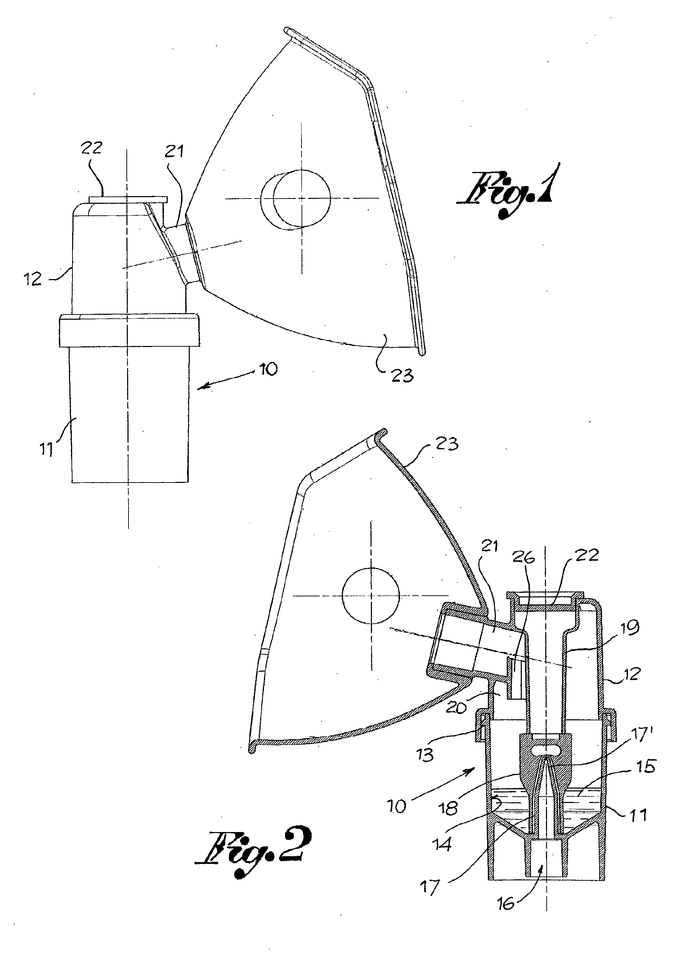

Fig. 1 shows an external view of the nebuliser complete with an accessory in the shape of a mask;
Fig. 2 shows an axial view of the nebuliser shown in Fig. 1;
Fig. 3 shows a view from below of an upper part of the nebuliser with the non-spill system;
Figs. 4 and 5 show two different sectional views according to the arrows B-B and C-C, respectively, in Fig. 3; and
Fig. 6 shows the nebuliser inclined to highlight the effectiveness of the non-spill system.

The illustrated nebuliser has a body 10 comprised of a lower element 11 and an upper element 12 fixed together disjointedly by means of a screw coupling 13, for example.

The lower element 11 delimits a chamber 14 designed to contain the medication to nebulise 15 and presents a tube 16 for entry of the nebulising air from a compressor - not shown.

The air tube 16 has a part 17 that rises in the chamber 14 above the level of liquid medication 15 and which terminates in a conical portion having orifice 17' at the summet. On the rising part of the air pipe 16 is a nebulising nozzle 18, commonly referred to as a "baffle", the structure and action of which are well known and do not require a detailed description here.

The upper element 12 of the body of the nebuliser 10 has a central through tube 19 and around it an annular tube 20 with a lateral opening 21. The central tube 19 is opened inferiorly towards the nebulising nozzle 18, whereas superiorly it may remain open or it can be closed by a stopper 22 or be fitted with an intake valve as required. The annular tube 20 allows the nebulised medication to rise from the nozzle 18 to the opening 21 to which it is possible to connect a mask 23 or a mouthpiece, or a paediatric nosepiece or an adult nosepiece.

Without the mask, mouthpiece or nosepiece, the opening 21 can be closed by means of a stopper to protect the inside of the nebuliser.

Optionally, instead of the stopper 22 for the central tube 19 there may be envisaged a one-way inlet valve - not shown here - allowing additional flow of air into the nebuliser for a different, known, method of nebulisation.

The operating principle of the nebuliser described above is known. The medication 15 is sucked up by the Venturi effect created by the inflow of air through the tube 16. The air is drawn towards the upper part of the nozzle 18 and reaches the orifices where the air and medication come together and are broken down finely by the obstacle represented by the baffle. This system allows maximum nebulising efficiency and therefore effective use of the medication.

According to the invention, the nebuliser comprises a non-spill system. This consists of an extension 24 of the lowest part of the wall of the lateral opening 21, towards the inside of the annular tube 20, and of a septum 25 at the end of said extension 24. The septum 25 is virtually parallel to the outer surface of the central tube 19, and its opposing vertical edges 25' are joined to said surface as shown in Figs. 3-5. The septum extends in height from below said extension 24 to approximately the geometric axis of the opening 21 and delimits, with the adjacent surface of the central tube 24, a passage 26 conveying the medication towards the opening.

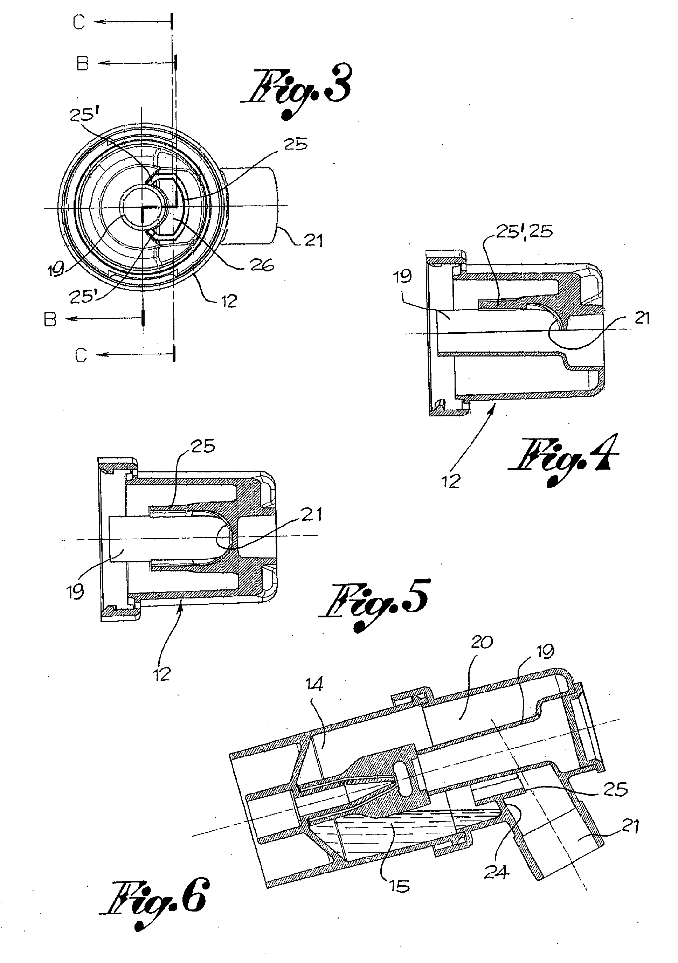

The septum 25 as arranged and configured herein forms a barrier preventing the medication from spilling out of the outlet when the nebuliser is inclined or placed on its side as shown for example in Fig. 6.

## Claims

1. A nebuliser for aerosol systems comprising a body (10) formed by two assembled elements (11, 12) and delimiting:
- a chamber (14) for the medication to nebulise;
- a tube (16) rising in said chamber for the intake of a nebulising flow of air under pressure and holding a nozzle (18);
- a central tube (19) open inferiorly towards the nozzle (18) and closed superiorly by a removable stopper; and
- an annular tube (20) round the central tube and extending in said chamber as far as a lateral opening (21) through which the nebulised medication is conveyed towards the user,
**characterised by** a non-spill system (24) between said lateral opening (21) and the central tube (19) to prevent the medication from spilling if the nebuliser is inclined or dropped.

2. A nebuliser as per claim 1, in which said non-spill system includes an extension (24) towards the inside of said annular tube (20) of a lower part of the wall of the lateral opening (21) and a septum (25) at the end of said extension (24) and substantially parallel to the outer surface of the central tube (19).

3. A nebuliser as per claim 2, in which the septum (25) has two opposing vertical edges (25') that are joined to the outer surface of said central tube (19).

4. A nebuliser as per claim 3, in which said septum extends in height from below the extension (24) to about the level of the geometric axis of the lateral opening (21) and delimits with the outer surface of the central tube a passage for the nebulised medication, but not the liquid medication if the nebuliser is inclined or placed on its side.
